# EUROPEAN PATENT APPLICATION

(11) **EP 2 573 259 A1**
(43) Date of publication of application: **27.03.2013**
(21) Application number: 12184501.0
(22) Date of filing: 14.09.2012
(51) Int. Cl.: D21C 3/04, C12P 7/10, C13K 1/02, C08H 8/00, C08B 37/00

(54) **Pretreatment method for producing water-soluble sugars from lignocellulosic material**

(30) Priority: 23.09.2011 FI 20115937
(71) Applicant: Chempolis Oy, 90660 Oulu (FI)
(72) Inventor: Sandqvist, Janne, 90660 Oulu (FI); Palola, Jaakko, 67700 Kokkola (FI); Rousu, Päivi, 90660 Oulu (FI)
(74) Representative: Puranen, Maija-Liisa

(57) **Abstract**

The invention relates to manufacturing hydrolyzable cellulose and further, if desired, sugars from lignocellulosic material by means of formic and performic acid treatment.

## Description

### Background of the invention

The invention relates to a pretreatment method for producing hydrolyzable cellulose from lignocellulosic material by using formic and performic acid treatment and, further, to manufacturing sugars from cellulose thus obtained. Peracid treatment results in cellulose suitable for hydrolysis, from which sugars, such as glucose, are then manufactured by means of enzymatic hydrolysis or other methods. A sugar product thus obtained is usable for manufacturing bioethanol and other industrial chemicals. The hydrolyzable cellulose according to the invention is suitable to be further processed not only into sugars but also into other products, for instance into various cellulose derivatives.

Publication US 2010/0240112 A1 (Juha Anttila et al.) discloses treatment of lignocellulose-containing biomass with organic acids (formic acid and/or acetic acid), which gives a carbohydrate fraction with improved hydrolyzability, after which the carbohydrate fraction is subjected to enzymatic hydrolysis for producing sugars, such as glucose. The acid treatment is typically performed at a temperature of 60 to 220 °C, preferably 100 to 180 °C, for example 130 to 170 °C. The method may, after acid cooking, additionally comprise a further treatment step where the reaction mixture is heated for example at a temperature of 100 to 180 °C (residence time e.g. 10 min to 24 h).

Publication US 2010/0285553 A1 (Michel Delmas, Compagnie Industrielle de la Matiere Vegetale CIMV) discloses a method for pre-teating a lignocellulosic plant raw material by using formic acid cooking at a temperature of 95 to 110°C. A product/products are obtained which can be hydrolyzed and fermented for producing bioethanol.

It was observed, however, that using mere formic acid or a mixture of formic acid and acetic acid in accordance with the prior art did not provide sufficiently good hydrolyzability for pulp.

The use of peracetic acid as a pretreatment method prior to enzymatic hydrolysis of biomass (bagasse) is disclosed in publication Zhao et al., Effect of several factors on peracetic acid pretreatment of sugarcane bagasse for enzymatic hydrolysis, Journal of Chemical Technology and Biotechnology, 82 (2007) 1115-1121. It is stated in the publication that peracetic acid treatment may efficiently improve enzymatic hydolyzability of bagasse by removing hemicellulose and lignin.

Dosages and amounts of peracetic acid and hydrogen peroxide used in known peracid methods are, however, high, which results in high peracid / hydrogen peroxide costs.

The effect of residual lignin on hydrolyzability of softwood cellulose pretreated by steam explosion has also been studied in publication Pan, X. et al., Strategies to Enhance the Enzymatic Hydrolysis of Pretreated Softwood with High Residual Lignin Content, Applied Biochemistry and Biotechnology 121-124 (2005), 1069-1079. It was noted that residual lignin affects enzymatic hydrolysis of cellulose by two different mechanisms: by forming a physical barrier to a "cellulase attack" or by binding cellulases, preventing thus their activity.

The use of performic acid for improving hydrolyzability of cellulose has not been described or suggested in the prior art. The use of performic acid is only known in manufacturing chemical paper pulp.

WO publication 97/26403 (Esa Rousu Consulting Oy) describes a method for manufacturing cellulose intended for manufacturing paper (or viscose) from nonwood-material by two-step formic acid cooking, whereby the second step uses hydrogen peroxide as an additive. The first step is performed at a temperature of over 100 °C, preferably at a temperature of 105 to 125 °C, the second step being performed at a temperature of 70 to 90 °C.

WO publication 98/20198 (Chempolis Oy) describes a method for manufacturing chemical pulp intended for manufacturing paper by single-step formic acid cooking by using performic acid for washing pulp. The performic acid is manufactured in situ or immediately before use by adding hydrogen peroxide or ozone to formic acid. The amount of hydrogen peroxide to be added is 0.01 to 1.5%, calculated from the cellulosic raw material. Performic acid washing is performed when the consistency of the pulp is 10 to 50%, preferably 20 to 35%. Formic acid cooking is performed at a temperature of over 85 °C, most preferably at a temperature of 110 to 125 °C. Performic acid treatment is performed at a temperature of 50 to 90 °C, preferably at a temperature of 60 to 80 °C. The duration of the performic acid treatment is stated to be the residence time of pulp in a typical washing step. According to Example 1, the duration of the performic acid treatment was 7 minutes.

US patent 6 866 749 B2 (Michel Delmas, Compagnie Industrielle de la Matiere Vegetale) describes the use of a mixture of peracetic acid and performic acid for bleaching chemical paper pulps.

### Brief description of the invention

An object of the invention is thus to provide a method for manufacturing hydrolyzable cellulose and further for manufacturing sugars so as to enable the aforementioned problems to be solved. The object of the invention is achieved by a method characterised by what is stated in the independent claims. The preferred embodiments of the invention are disclosed in the dependent claims.

The invention is based on the idea that pretreatment of cellulosic material for improving hydrolyzability is carried out with formic acid and performic acid under given conditions and for a given period of time. The hydrolyzability of the obtained pulp into sugars is excellent with a low enzyme dosage, despite the fact that the lignin and hemicellulose contents were not in all cases as low as in methods previously known in the field for manufacturing hydrolyzable chemical pulp.

Advantages of the method and the arrangement according to the invention include providing hydrolyzable cellulose with an inexpensive pretreatment method and being able to use a low enzyme dosage in hydrolysis without the lignin and hemicellulose contents of the cellulose pulp being critical in the hydrolysis.

### Detailed description of the invention

An object of the invention is a method for manufacturing cellulose and/or a sugar product from lignocellulosic material.

The method according to the invention is characterized by
(a) subjecting the lignocellulosic material to fractionation by formic acid cooking at a formic acid content of 70 to 98% at a temperature of 110 to 160°C;
(b) bringing the cellulose pulp fraction thus obtained to a consistency of 10 to 40% and washing it with formic acid;
(c) treating the thickened and washed cellulose pulp at 50 to 90 °C with performic acid made by adding hydrogen peroxide to formic acid in the amount of 0.5 to 3%, calculated from dry pulp, and by allowing the formic acid and hydrogen peroxide to react into performic acid for 1 to 10 minutes before adding the performic acid to the thickened and washed pulp;
   whereby performic acid treatment is performed until the performic acid has been substantially consumed, and the treatment is subsequently continued by allowing the reaction mixture to react at the same temperature;
(d) washing the cellulose pulp, which has been treated with performic acid, with formic acid whose content is 70 to 98%;
(e) treating the cellulose pulp thus obtained with formic acid whose content is 2 to 20% at a temperature of 60 to 150 °C;
(f) recovering the obtained cellulose; and
(g) if desired, hydrolyzing the obtained cellulose into monosaccharides and oligosaccharides, whereby a sugar product is obtained.

Steps (a) to (e) relate to pretreatment for obtaining hydrolyzable cellulose while step (g) relates to hydrolysis of the cellulose thus obtained into sugars.

In the context of the present invention, hydrolyzable cellulose refers to cellulose pulp which is, with regard to its properties, particularly suitable for producing monosaccharides and oligosaccharides, such as glucose, with enzymatic hydrolysis. Manufacture of hydrolyzable cellulose aims at a low hemicellulose content, for example, as distinct from a higher hemicellulose content that is an aim for chemical paper pulp and that has a positive effect on the binding properties of paper pulp. Further, the lowest possible kappa number that is an aim for chemical paper pulp is not necessary in manufacturing hydrolyzable cellulose.

In step (a) of the method according to the invention, the lignocellulosic material is subjected to fractionation with formic acid cooking by using formic acid whose content is 70 to 98%, preferably 75 to 85% (the rest being water). The cooking reagent may also contain small amounts of acetic acid, the amount being less than 30%, preferably less than 15%, particularly preferably less than 1%, calculated from the weight of the total cooking reagent. The cooking is performed at a temperature of 110 to 160 °C, preferably 125 to 135 °C. The cooking time is 5 to 80 minutes, preferably 20 to 60 minutes. The ratio of lignocellulosic material to liquid is 1:4 to 1:6. The cooking is performed at a slight overpressure of 2 to 6 bar, preferably 2 to 4 bar.

From the fractionation, a cellulose-containing solid matter fraction is recovered which is further treated in steps (b) to (f) of the method according to the invention.

After the formic acid cooking, the obtained cellulose pulp fraction is thickened in step (b) to a consistency of 10 to 40% with conventional methods by using a screw or pressure press, a vacuum press or the like.

Subsequently, the obtained cellulose pulp is washed in step (b), typically with the same reagent that was used in the cooking (formic acid whose content is 70 to 98%). The extraction time in washing step (b) may be 30 minutes, for example, and the temperature 30 to 90 °C, preferably 60 to 90 °C.

The thickened and washed pulp is treated with performic acid in step (c). The performic acid is made by adding hydrogen peroxide to formic acid, the amount of hydrogen peroxide being 0.5 to 3%, preferably 0.5 to 2%, calculated from dry pulp, and by allowing the formic acid and hydrogen peroxide to react into performic acid for 1 to 10 minutes, preferably for 3 to 7 minutes, before adding the performic acid to the thickened and washed pulp. The performic acid is produced at a temperature of 50 to 90 °C, preferably 65 to 75 °C.

The performic acid treatment is performed at a temperature of 50 to 90 °C, preferably at 65 to 75 °C. The performic acid is allowed to influence the pulp until the performic acid has been substantially consumed, the treatment being subsequently continued by allowing the reaction mixture to react at the same temperature. It was observed that hydrolyzability was improved when the treatment was continued although no more peracid was left in the reaction mixture.

In one embodiment of the invention, the performic acid treatment of step (c) is carried out in a separate reactor. In this case, the consistency of the pulp may typically be 10 to 15%.

In another embodiment of the invention, the performic acid treatment of step (c) is carried out in a washer in connection with washing the pulp. In this case, the consistency of the pulp may typically be 20 to 35%.

The total time of the performic acid treatment in step (c) is 30 to 200 minutes, preferably 150 to 200 minutes.

After the performic acid treatment, the pulp is rewashed with formic acid in step (d). The same reagent that was used in cooking (formic acid whose content is 70 to 98%) may be used for the washing. The extraction time in washing step (b) may be 15 minutes, for example, and the temperature 30 to 90 °C, preferably 60 to 90 °C.

In step (e), de-esterification of the pulp is performed by treating the pulp with diluted formic acid (content 2 to 20%, preferably 2 to 6%) at a low consistency (1 to 5%) at a temperature of 60 to 150 °C (preferably 80 to 90 °C). The treatment time in step (e) is 0.5 to 24 hours, typically 12 to 20 hours. In the de-esterification step, organic acids which were bound to the pulp in a chemically esterified form in the cooking step are released from the cellulose pulp.

After this, the pulp is subjected to conventional filtering and water washing steps.

The result is hydrolyzable cellulose with a kappa number of 3 to 55 and a hemicellulose content of 0.5 to 7%.

It was observed that hydrolyzability of the cellulose obtained in accordance with the invention into sugars is excellent with a low enzyme dosage. It has also been observed that peracid treatment according to the invention does not essentially affect the lignin and hemicellulose contents.

Owing to its good hydrolyzability, the cellulose obtained in this way is very well suited for manufacturing water-soluble monosaccharides and oligosaccharides, such as glucose.

The cellulose pulp obtained in step (g) of the method according to the invention is, if desired, hydrolyzed into water-soluble monosaccharides and oligosaccharides, whereby a sugar product is obtained. The sugar product is preferably glucose. Preferably, enzymatic hydrolysis is used, but also other methods may be used for manufacturing sugars.

The method according to the invention may also comprise an additional step where the monosaccharides and oligosaccharides are fermented into ethanol.

In one embodiment of the invention, hydrolysis into sugars and fermentation into ethanol are performed successively in separate operations.

In another embodiment of the invention, hydrolysis into sugars and fermentation into ethanol are performed simultaneously in the same operation.

The invention also relates to the use of the cellulose obtained with the method according to the invention for manufacturing sugars and other products.

The invention further relates to the use of monosaccharides and oligosaccharides, such as glucose, obtained with the method according to the invention for manufacturing bioethanol by fermentation and also for manufacturing other industrial chemicals.

In the cooking step (a) of the method according to the invention, the lignin and hemicelluloses contained by the lignocellulosic pulp dissolve for the most part into the cooking liquor. The cooking acids may be recovered from the cooking liquor by means of known methods. In addition, furfural, acetic acid, formic acid, chemicals and biofuels may be produced from the lignin and hemicelluloses (C5 sugars, e.g. xylose) obtained as a by-product in the cooking step and possibly in other method steps.

The lignocellulosic material used as the starting material of the method may be any lignocellulosic plant material. It may be wood material, such as softwood or hardwood, such as eucalyptus or acacia. It may also be non-wood material based on herbaceous plants, bast fibres, leaf fibres or fruit seed fibres. Examples of usable materials based on herbaceous plants include straw, such as cereal straw (wheat, rye, oat, barley, rice), reeds, such as reed canary grass, common reed, papyrus, sugar cane, i.e. bagasse, and bamboo, as well as grasses, such as esparto, sabai and lemon grass. Examples of bast fibres include flax, such as stalks of common flax and stalks of oil flax, stalks of cassava, hemp, East Indian hemp, kenaf, jute, ramie, paper mulberry, gampi fibre and mitsumata fibre. Examples of leaf fibres include abaca and sisal. Examples of fruit seed fibres include cottonseed hairs and cotton linter fibres, kapok and coir fibre.

Herbaceous plants growing in Finland and usable in the present invention include common reed, reed canary grass, timothy, cocksfoot, yellow sweet clover, smooth brome, red fescue, white sweet clover, red clover, goat's rue and alfalfa.

Particularly preferably, biomass based on herbaceous plants, such as cereal straw, is used. In an embodiment, biomass based on annual herbaceous plants is used. In another embodiment, biomass based on perennial non-woody plants is used. In accordance with the invention, lignocellulose-containing waste material from industry or agriculture, including empty fruit bunch (press residues of oil palm) and above-mentioned cereal straw, may also be used.

### Examples 1 to 5

In the following, the invention will be described with reference to illustrating but non-restrictive examples.

In Examples 1 to 3, the effect of hydrogen peroxide amounts on hydrolyzability was examined. In Examples 4 to 5, the effect of the peracid treatment time on hydrolyzability was examined.

Per cent contents in the examples and in the whole of the description and claims are percentages by weight (w-%) unless otherwise stated.

In the examples, the properties of the obtained cellulose pulps were determined by using the following methods:

The kappa number (lignin content) was determined by using standard method ISO 302:2004.

The proportion of pentosan sugars (xylan) in the dry matter was determined on the basis of standard method SCAN:C4-61, and the analyses relating thereto were made with HPLC.

Determination of the ash content was based on standard method ISO-1762:2001.

The hydrogen peroxide content was determined by titrating the hydrogen peroxide with ammonium ceric sulphate in acidic conditions by using ferroin indicator. The performic acid content was determined from the same solution after potassium iodide addition by titrating with sodium thiosulphate and using ammonium molybdate as a catalyst and starch as an indicator.

Enzymatic hydrolysis of pulps was performed by using a commercial cellulase product as a standard dose, calculated from the dry weight of the cellulose pulp sample. The cellulase product contained cellulases produced with mould fungus Trichoderma reesei as the main component and it contained all required activities for hydrolyzing cellulose (cellulase activity being about 132 FPU ["Filter Paper Unit"], determined by standard method NREL/TP-510-42628, January 2008). The enzyme dosage in Examples 1 to 3 was 5 to 6 FPU / 1 g of dry cellulose and in Examples 4 to 5 about 5 FPU / 1 g of dry cellulose. The duration of the hydrolysis test was 3 to 7 days. Hydrolyzability was examined by determining the progress of the hydrolysis on the basis of the solid matter (%) and/or by determining the glucose yield.

The solid matter test was based on gravimetric determination and performed as follows: (1) a standard amount of solid cellulose pulp was dosed to a mixing bottle; (2) enzymatic hydrolysis was performed in the above manner, whereby the solid cellulose was hydrolyzed in the above manner into water-soluble sugars, i.e. monosaccharides and oligosaccharides, which resulted in a decrease in the solid matter; (3) the remaining solid matter was separated by filtering; (4) the decrease in the solid matter, which is proportional to the progress of the enzymatic hydrolysis, was determined; (5) the maximum decrease in the solid matter with a high dose of enzyme (e.g. with an enzyme amount three times larger than in actual hydrolysis tests) was determined; (6) the maximum decrease in the solid matter was compared with the decrease in the solid matter in a test sample, whereby

progress of hydrolysis (%) = (decrease in solid matter in test sample) / (maximum decrease in solid matter).

The glucose yield was calculated as follows: (1) the cellulose content of the sample was estimated on the basis of the kappa number, pentose content (C5 sugar content) and ash content; (2) the amount of glucose obtained from the enzymatic hydrolysis was divided by the estimated cellulose content; and (3) the obtained ratio was multiplied by the ratio of the molar mass of the cellulose unit to the molar mass of glucose (162/180).

### Example 1

Empty fruit bunch was subjected to formic acid cooking at a temperature of 120.5 °C by using 82-% formic acid. The ratio of raw material to liquid was 1:6. The cooking time was 25 minutes.

After the cooking, the pulp was filtered to a dry a matter content of 12% and washed, at the same time, with the formic acid reagent that had been used in the cooking. The extraction time in the wash was 30 minutes.

A performic acid reagent was prepared by adding hydrogen peroxide to a small amount of formic acid at 70 °C and by allowing the mixture to react for 5 minutes.

The filtered and washed pulp (at a consistency of 10 to 12%) was treated with the performic acid reagent obtained in this way at 70 °C for 65 minutes. The amount of hydrogen peroxide used for the treatment varied in the range of 0.5 to 3.6% per supplied dry pulp. A reference test was also performed in which there was no hydrogen peroxide at all (0%).

Subsequently, the pulp was washed with the same acid as above by using an extraction time of 15 minutes in the wash.

The washed pulp was subjected to de-esterification by reacting the pulp at a consistency of 2% with diluted formic acid (content 2 to 6%) at a temperature of 85 °C for 16 hours.

After this, the pulp was filtered and washed with water.

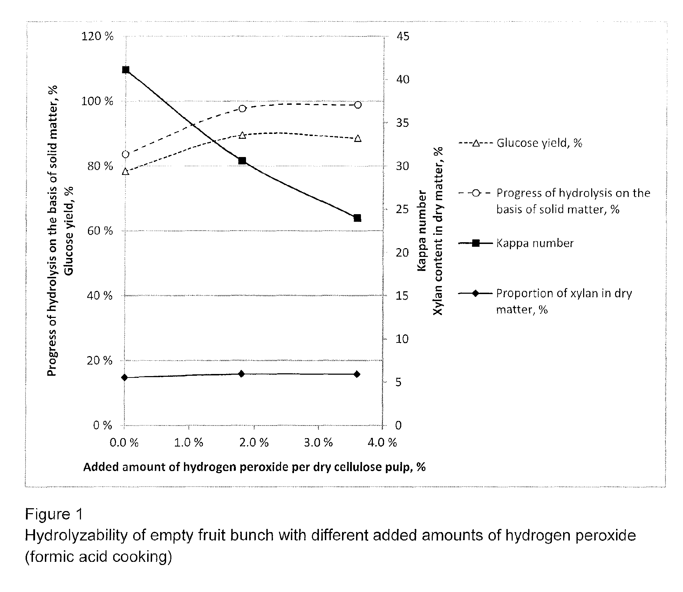

From the cellulose pulps obtained in this way and treated with different amounts of hydrogen peroxide, the proportion of xylan, the kappa number and the enzymatic hydrolyzability were determined in the above manner (duration of the hydrolysis being 5 days). The results are shown graphically in Figure 1.

It can be seen in Figure 1 that increasing the amount of hydrogen peroxide, i.e. the amount of performic acid generated, essentially improves the hydrolyzability of pulp but does not substantially change the content of xylan (pentose sugars) in the cellulose pulp.

### Example 2

Formic acid and performic acid treatments similar to those in Example 1 were performed by using stalks of cassava as raw material. The cooking conditions were the same, except that the cooking temperature was 130 °C and the cooking time 60 minutes.

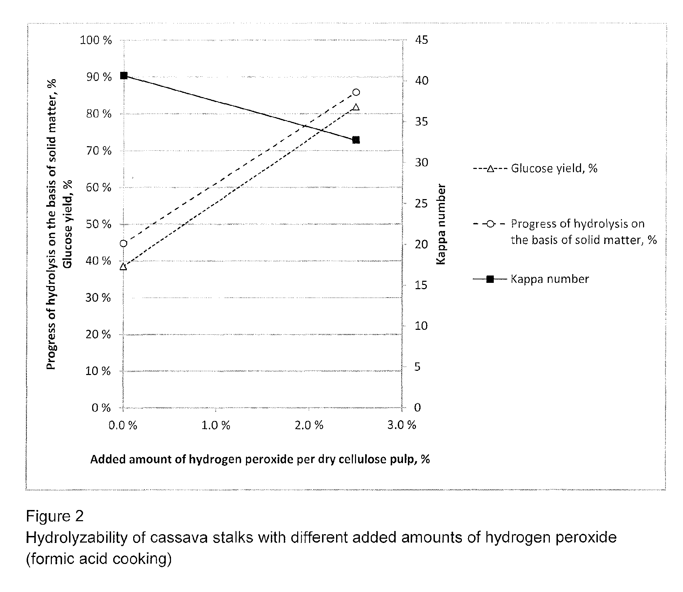

From the cellulose pulps obtained in this way and treated with hydrogen peroxide amounts of 0% and 2.6%, the kappa number and hydrolyzability were determined in the same way as above. The results are shown graphically in Figure 2.

The results are similar to those in Example 1. Increasing hydrogen peroxide and performic acid essentially lowers the kappa number (lignin content) and, at the same time, improves hydrolyzability.

### Example 3 (reference test)

The effects of hydrogen peroxide addition and of peracids were further studied by carrying out reference cooking with an acid mixture that contained equal amounts of formic acid and acetic acid (total acid amount being 82%, of which 50% was formic acid and 50% acetic acid). Stalks of cassava were used as raw material for the cooking. The cooking was performed at a temperature of 160 °C, the cooking time being 30 minutes.

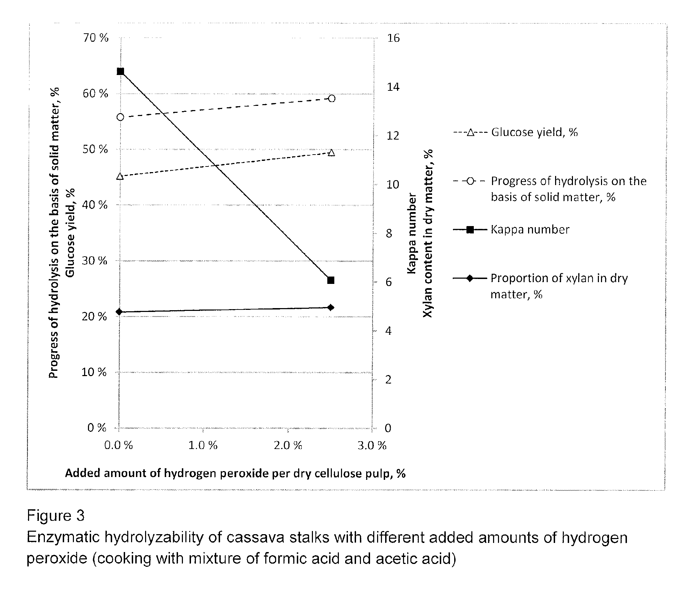

From the cellulose pulps obtained in this way and treated with hydrogen peroxide amounts of 0% and 2.6%, the kappa number, the xylan content and the hydrolyzability were determined in the same way as above. The results are shown graphically in Figure 3.

It can be seen from the results that the addition of hydrogen peroxide and performic and peracetic acids essentially lowers the kappa number but does not have an improving effect on the hydrolyzability of cellulose, nor does it affect the xylan content.

Thus, using a large amount of acetic acid in cooking in addition to formic acid did not give, with regard to hydrolyzability, cellulose of as high quality as that obtained from mere formic acid cooking.

### Example 4

Eucalyptus chips were subjected to formic acid cooking by using acetic acid as an additional cooking chemical (acidic composition of the cooking acid: 70% formic acid and 12% acetic acid). The cooking temperature was 150 °C, the cooking time being 60 minutes.

Peracid treatment was performed by adding hydrogen peroxide in the amount of 1.6% per supplied dry pulp, the time of the peracid treatment being 65 minutes or 180 minutes.

The treatment also included washing and de-esterification steps corresponding to those in Example 1.

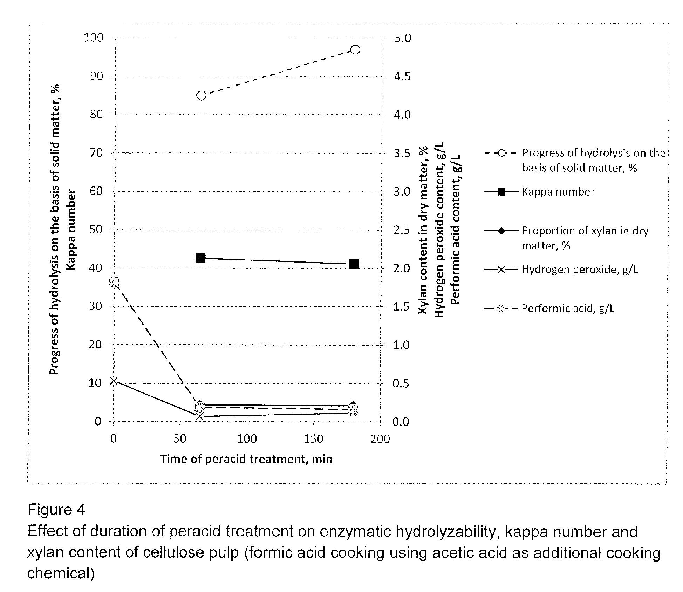

The process included determining the kappa number, the xylan content and the progress of hydrolysis on the basis of solid matter as well as the glucose yield and the hydrogen peroxide content of the reaction mixture. The results are shown graphically in Figure 4. The glucose yield (not shown in the figure) in a 7-day hydrolysis test was 82% (duration of the peracid treatment 65 minutes) and 100% (duration of the peracid treatment 180 minutes).

The results show that as the peracid treatment time gets longer, hydrolyzability improves, whereas the kappa number and xylan content remain substantially unchanged. It can also be observed in Figure 4 that hydrolyzability was improved when the treatment is continued even after there is substantially no hydrogen peroxide / peracid left in the reaction mixture.

### Example 5

Formic acid cooking with 82-% formic acid was performed for the same raw material as in Example 4. The cooking temperature was 130 °C, the cooking time being 15 minutes.

Peracid treatment was performed by adding hydrogen peroxide in the amount of 1.6% per dry pulp, the durations of the peracid treatment being the same as in Example 4.

The treatment also included washing and de-esterification steps corresponding to those in Example 1.

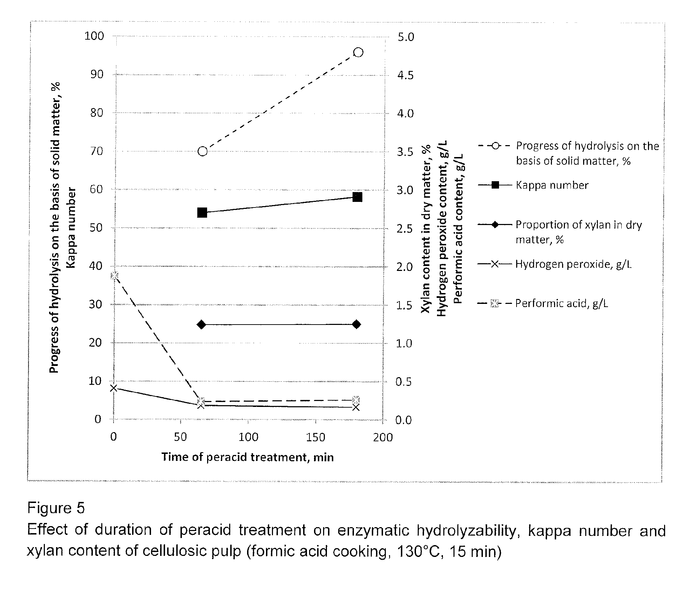

The process included determining the kappa number, the xylan content and the progress of hydrolysis on the basis of solid matter as well as the glucose yield and the hydrogen peroxide / peracid content of the reaction mixture. The results are shown graphically in Figure 5. The glucose yield (not shown in the figure) in a 7-day hydrolysis test was 68% (duration of the peracid treatment 65 minutes) and 93% (duration of the peracid treatment 180 minutes).

The results show that as the peracid treatment time gets longer, hydrolyzability on the basis of solid matter is improved, the kappa number is increased only somewhat and the xylan content remains substantially unchanged. Hydrolyzability is improved when the treatment is continued even after there is substantially no hydrogen peroxide / peracid left in the reaction mixture.

It will be apparent to one skilled in the art that as technology advances, the basic idea of the invention may be implemented in many different ways. The invention and its embodiments are thus not restricted to the examples described above but may vary within the scope of the claims.

## Claims

1. A method for manufacturing cellulose and/or a sugar product from lignocellulosic material, **characterized by**
(a) subjecting the lignocellulosic material to fractionation by formic acid cooking at a formic acid content of 70 to 98% at a temperature of 110 to 160°C;
(b) bringing the cellulose pulp fraction thus obtained to a consistency of 10 to 40% and washing it with formic acid;
(c) treating the thickened and washed cellulose pulp at 50 to 90 °C with performic acid made by adding hydrogen peroxide to formic acid in the amount of 0.5 to 3%, calculated from dry pulp, and by allowing the formic acid and hydrogen peroxide to react into performic acid for 1 to 10 minutes before adding the performic acid to the thickened and washed pulp;
whereby performic acid treatment of the cellulose pulp is performed until the performic acid has been substantially consumed, and the treatment is subsequently continued by allowing the reaction mixture to react at the same temperature;
(d) washing the cellulose pulp, which has been treated with performic acid, with formic acid whose content is 70 to 98%;
(e) treating the cellulose pulp thus obtained with formic acid whose content is 2 to 20% at a temperature of 60 to 150 °C;
(f) recovering the obtained cellulose; and
(g) if desired, hydrolysing the obtained cellulose into water-soluble monosaccharides and oligosaccharides, whereby a sugar product is obtained.

2. The method according to claim 1, **characterized by** the cooking time of step (a) being 5 to 80 minutes.

3. The method according to claim 1 or 2, **characterized by** the ratio of lignocellulosic material to liquid in step (a) being 1:4 to 1:6.

4. The method according to any one of the preceding claims, **characterized by** the performic acid treatment in step (c) being performed in a separate reactor.

5. The method according to any one of claims 1 to 3, **characterized by** the performic acid treatment in step (c) being performed in a washer in connection with washing the pulp.

6. The method according to any one of the preceding claims, **characterized by** the total time of the performic acid treatment in step (c) being 30 to 200 minutes, preferably 150 to 200 minutes.

7. The method according to any one of the preceding claims, **characterized by** the treatment time in step (e) being 0.5 to 24 hours.

8. The method according to any one of the preceding claims, **characterized by** the kappa number of the cellulose pulp obtained from step (e) being 3 to 55.

9. The method according to any one of the preceding claims, **characterized by** the hemicellulose content of the cellulose pulp obtained from step (e) being 0.5 to 7%.

10. The method according to any one of the preceding claims, **characterized by** the obtained sugar product being glucose.

11. The method according to any one of the preceding claims, **characterized by** the method further comprising a step where the monosaccharides and oligosaccharides of the obtained sugar product are fermented into ethanol.

12. The method according to any one of claims 1 to 10, **characterized by** performing simultaneously fermentation of monosaccharides and oligosaccharides into ethanol in step (g).

13. Use of cellulose obtained with the method according to any one of claims 1 to 10 for manufacturing sugars and other products.

14. Use of monosaccharides and oligosaccharides obtained with the method according to any one of claims 1 to 10 for manufacturing bioethanol and other industrial chemicals.
